(19) Europäisches Patentamt
European Patent Office
Office européen des brevets

(11) Publication number: **0 073 540**
**B1**

(12) **EUROPEAN PATENT SPECIFICATION**

(45) Date of publication of patent specification: **24.04.85**

(51) Int. Cl.[4]: **C 07 C 121/66,** C 07 C 120/00

(21) Application number: **82201027.8**

(22) Date of filing: **16.08.82**

(54) **Preparation of tetracyanoquinodimethane.**

(30) Priority: **31.08.81 US 297881**

(43) Date of publication of application:
**09.03.83 Bulletin 83/10**

(45) Publication of the grant of the patent:
**24.04.85 Bulletin 85/17**

(84) Designated Contracting States:
**AT BE CH DE FR GB IT LI LU NL**

(56) References cited:
**US-A-3 115 506**

(73) Proprietor: **THE PROCTER & GAMBLE COMPANY**
**301 East Sixth Street**
**Cincinnati Ohio 45202 (US)**

(72) Inventor: **Crawford, Robert James**
**397 Circlewood Lane**
**Cincinnati Ohio 45215 (US)**

(74) Representative: **Gibson, Tony Nicholas et al**
**Procter & Gamble (NTC) Limited Whitley Road**
**Longbenton Newcastle upon Tyne NE12 9TS**
**(GB)**

**EP 0 073 540 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention involves a new method for the conversion of 1,4-bis(dicyanomethylene)-cyclohexane (BDCC) to 7,7,8,8-tetracyanoquinodimethane (TCNQ) using chlorine and an aromatic amine, especially pyridine.

7,7,8,8-tetracyanoquinodimethane is a unique organic molecule because of its ability to accept electrons from donor substances. It is one of the most powerful electron acceptors known. This property has stimulated extensive research interest during the last decade. TCNQ can be combined with strong electron donors to form crystalline complexes that have electrical conductivities approaching those of metals. Various research groups are now working with TCNQ complexes in order to develop organic metals and organic semi-conductors.

TCNQ is also an essential catalytic ingredient in the alpha-chlorination of fatty acids, as described in U.S. Patent 4,148,811. The compound may find still other uses in chemical processing as research continues.

All practical chemical syntheses of TCNQ require the use of BDCC as the immediate precursor to TCNQ. The transformation of BDCC to TCNQ requires an oxidation, since the net change involves the removal of four hydrogen atoms and formation of two olefinic double bonds. Several oxidizing agents have been reported to accomplish this conversion; however, the best method to date has been the original technique of halogenation and dehydro-halogenation, discovered by DuPont, using bromine and pyridine. In this reaction, a suspension of BDCC in acetonitrile is mixed with two equivalents of bromine and then four equivalents of pyridine are added over a fifteen minute period. When the reaction is complete, TCNQ has precipitated from the acetonitrile along with a substantial portion of four equivalents of pyridine hydrobromide by-product. In order to separate the latter from the TCNQ, the entire reaction mixture must be diluted with at least its own volume of water. The pyridine hydrobromide dissolves in the water, while TCNQ remains undissolved. The TCNQ is then collected by filtration and washed with additional water to remove acetonitrile and any remaining pyridine hydrobromide.

Unfortunately, when TCNQ is isolated using an aqueous work-up procedure it is always contaminated with another by-product of unknown constitution. This impurity is an inert, non-volatile, high melting brown powder, and is probably a polymeric derivative of BDCC. It can be separated cleanly, and remains as a residue, when TCNQ is sublimed. This impurity is usually present to the extent of approximately 10% by weight in TCNQ made by the DuPont process. The impurity can also be removed by recrystallizing the crude TCNQ from acetonitrile. Since sublimation is only practical on a small scale, recrystallization is the purification method used in the DuPont synthesis. Recrystallization of crude TCNQ requires approximately 50—80 ml recrystallization solvent (acetonitrile) per gram TCNQ, and affords pure TCNQ in 80% yield based on BDCC. Thus, while the yield of crude TCNQ from the DuPont process is reported to be high, significant losses are incurred in subsequent purification.

Direct substitution of chlorine for bromine in the oxidation of BDCC to TCNQ would be attractive for economic reasons. However, this is not a useful modification because TCNQ has been found to react with chlorine to form a α, α'-dichloro-p-phenylenedimalononitrile (DCPDM) according to the following schematic

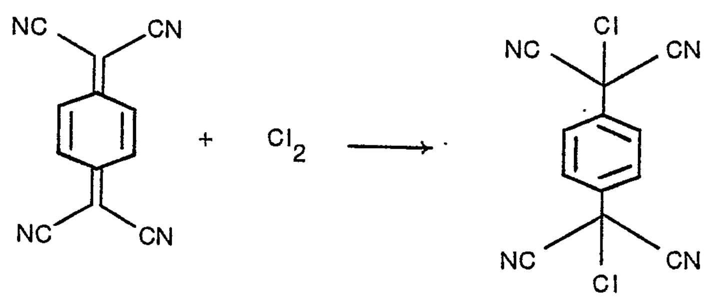

This reaction has been found to be rapid at room temperature and, unfortunately, is catalyzed by chloride ion. No analogous reaction occurs when bromine is used as the halogen. Substition of chlorine for bromine in the DuPont process causes chlorine to be present in excess throughout the reaction, and a large quantity of chloride ion is generated in the form of pyridine hydrochloride. As a consequence, TCNQ has ample opportunity, as it is formed, to react with excess chlorine, catalyzed by the chloride ion by-product. Results of attempts to substitute chlorine for bromine in this manner have verified this hypothesis. TCNQ is isolated in approximately 45% yield, and is heavily contaminated with DCPDM and other, unknown byproducts.

Chlorine excess, and DCPDM formation, can be avoided by reversing the mode of addition, i.e. all the pyridine can be added to the BDCC first, followed by slower addition of chlorine. Unfortunately, this technique results in substantial increases in the polymeric BDCC by-product described above. If chlorine addition is started immediately after addition of pyridine, the polymeric impurity constitutes 25% of the crude product, rather than 10%. If BDCC is allowed to contact pyridine for an hour before chlorine addition, the polymeric by-product (brown powder) constitutes 69% of the resulting reaction products.

Brief Description of the Drawing

The Figure illustrates the pyridine addition schedule employed in the practice of a preferred embodiment of this invention. An "idealized" addition rate schedule for pyridine, 12, is illustrated, assuming simultaneous equimolar addition of chlorine and pyridine were attempted. This "stoichiometric" curve thus also represents the chlorine addition schedule, for reference purposes. Also illustrated is the actual, preferred addition rate employed, 10, in which 1) pyridine addition is delayed for 15 seconds after chlorine addition is started; and 2) the pyridine addition rate is doubled when the reaction is 80% complete, so that pyridine addition is complete at 90% of reaction completion.

Disclosure of the Invention

This invention involves a process for the oxidation, by a halogenation and dehydrohalogenation reaction, of a sustituted or unsubstituted 1,4-bis(dicyanomethylene)-cyclohexane to the corresponding 7,7,8,8-tetracyanoquinodimethane. While accomplished in a single step, this reaction follows the general schematic:

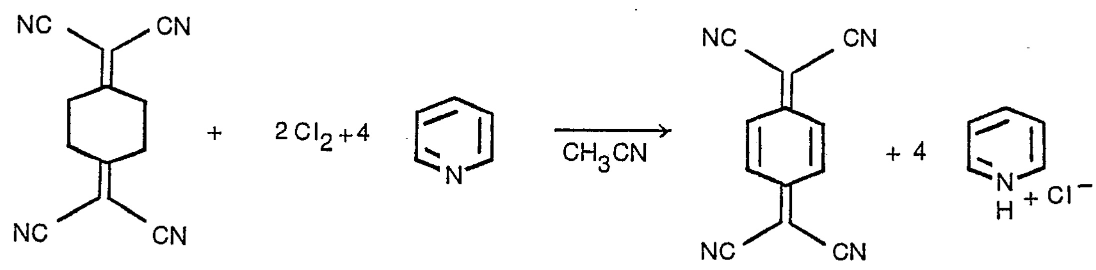


It thus involves the introduction of chlorine and an aromatic amine into the reaction mixture (generally BDCC in a suitable solvent, as described hereinafter) in a 4:2:1 amine:chlorine:BDCC stoichiometric ratio. The reaction is relatively rapid and exothermic.

The present invention provides a technique for the simultaneous use of chlorine and an aromatic amine, especially pyridine, in the oxidation of BDCC to TCNQ via approximately simultaneous addition of both reagents. As described before, the conversion of BDCC to TCNQ with chlorine and aromatic amine is subject to the constraint that neither chlorine nor amine can be present in large excess during the course of the reaction. In principle, this condition can be realized by employing a simultaneous addition of chlorine and amine to BDCC. In practice, however, this is difficult, and it is likely that at any given moment either chlorine or amine will be in slight excess, with consequent degradation of reaction quality.

It has now been discovered that chlorine in slight excess in this reaction is harmless, and leads to no detectable formation of DCPDM contaminant. It has also been discovered that an excess of amine, particularly during the early portion of the reaction process, is quite deleterious, and leads to significantly increased formation of the (presumed) polymeric BDCC by-product. In addition, the amount of impurity formed is roughly proportional to the extent that the amine is in excess.

Thus, in its broadest aspect, this invention provides a process for the halogenation and dehydrohalogenation of a substituted or unsubstituted 1,4-bis(dicyanomethylene)cyclohexane to the corresponding 7,7,8,8-tetracyanoquinodimethane by reacting chlorine and an aromatic amine with the 1,4-bis(dicyanomethylene)cyclohexane in a substantially 4:2:1 amine:chlorine: 1,4-bis(dicyanomethylene)cyclohexane molar ratio, comprising simultaneously adding the chlorine and aromatic amine at substantially stoichiometric ratios to a reaction mixture containing the 1,4-bis(dicyanomethylene)cyclohexane, while maintaining chlorine in slight stoichiometric excess. The latter condition is easily satisfied by slightly delaying initiation of amine addition, so that, while chlorine and aromatic amine are added at stoichiometrically matching rates, chlorine will always be in slight excess.

However, it has also been discovered that amine excess at the end of the reaction is also beneficial. If all of the aromatic amine is present before the reaction is stoichiometrically complete, and chlorine addition continues, a vivid color change from dark brown to orange can be observed at the precise moment when the required amount of chlorine has been added. This color change resembles the endpoint of a titration, and indicates that the chlorine flow should be stopped immediately. This is particularly important and valuable because it is at the end of the reaction that TCNQ is most vulnerable to reaction with excess chlorine. If chlorine flow is continued for even a short time beyond this stoichiometric endpoint, a significant portion of the TCNQ will be lost by conversion to DCPDM.

In order to realize the benefits of excess chlorine during the majority of the reaction, and excess amine at the end, the present invention provides a process for chlorine and aromatic amine addition which uses two addition rates. Thus, this invention also provides a preferred process which further comprises a) increasing the addition of the amine beyond a stoichiometric rate when the reaction is 70—90% complete, so that a stoichiometric quantity of the amine is added prior to reaction completion; and b) stopping addition of the amine substantially immediately upon addition of a stoichiometric quantity of the

amine to the reaction mixture; followed by c) stopping addition of chlorine substantially immediately upon stoichiometric completion of the reaction. Stoichiometric completion of the reaction is preferably determined by the change of color of the reaction mixture described above. In a preferred process, chlorine is added at a substantially constant rate throughout the reaction, while pyridine is added at a matching rate through only about 80% of the reaction. Also, the start of pyridine addition is delayed for a short interval so that it will lag slightly behind the chlorine during this period. At about the 80% point, the pyridine rate is approximately doubled, and pyridine addition is thus completed at about 90% of reaction completion. In this way, the endpoint signifying the end of chlorine addition can be observed easily. Because the polymeric BDCC by-product is formed from a reaction between the amine and BDCC, and because the concentration of BDCC is too low at the 90% reaction point for this side reaction to be significant, the excess of pyridine during the last 10% of the reaction does not have a detrimental effect.

A preferred reagent addition schedule for a standard ten minute reaction is illustrated graphically in the Figure. The chlorine flow (equivalent to curve 12) is started at time zero, and the pyridine flow 10 is started at a stoichiometrically matching rate 15 seconds later. At 8 minutes, the pyridine rate is doubled, and the addition is completed at 9 minutes. The endpoint for chlorine addition is usually observed at 9.5—9.75 minutes. After chlorine flow is stopped, a slight reversion of the endpoint color is observed over a period of five minutes. The orange color is then restored by an additional brief addition of chlorine.

A major advantage of this preferred procedure derives from the greater solubility of pyridine hydrochloride in acetonitrile. Thus, in the process of this invention, pyridine hyrochloride does not co-precipitate with TCNQ at the end of the reaction, and the need for product isolation from water is completely obviated. Isolation of the TCNQ requires nothing more than simple filtration of the reaction mixture, and the TCNQ thus obtained has a purity of 99+%. Furthermore, this invention is able to provide essentially pure TCNQ in 86—87% yield, compared to the 80% yield, after recrystallization, of prior art processes.

Elimination of the aqueous work-up procedure has further economic benefits in addition to eliminating the need for product recrystallization. In commercial TCNQ synthesis, it would be desirable to recover and reuse both pyridine and acetonitrile. Recovery of these materials is considerably more difficult if they are dissolved in water. By the practice of this invention, recovery of pyridine and acetonitrile from the reaction filtrate is made extremely simple. Distillation of the filtrate provides acetonitrile ready for reuse. The distillation residue consists primarily of pyridine hydrochloride, which can be reconverted to pyridine by treatment with alkali, followed by distillation.

A lab-scale method of practicing the preferred process of this invention is illustrated in the following example.

Example I

A 500 ml round bottom 4-neck flask was fitted with a mechanical stirrer, thermometer, 5 mm I.D. gas introduction tube extending near the bottom of the flask, and a 50 ml burette positioned for liquid addition into an open neck. The flask was charged with 25.0 g (0.120 mole) of 1,4-bis(dicyanomethylene)cyclohexane and 300 ml of acetonitrile, and the burette was filled with 39.0 ml (0.483 mole) of pyridine. The gas tube was connected to a chlorine supply tank by way of a glass ball rotameter, calibrated for chlorine. The stirrer was started, and the contents of the flask were heated to 40—50°C by using a heating mantle. The mantle was removed, the gas tube was temporarily removed from the flask, and the chlorine flow was started at a true flow rate of 620 ml/min. (On a reaction of this scale, this flow rate has been found experimentally to supply 0.24 mole chlorine to the reaction in almost exactly 10 minutes; this corresponds to an "effective" flow rate of 550 ml/min.) When the gas flow had stabilized, the tube was reinserted into the reaction flask and a stopwatch suitable for timing the reaction was started at the same instant. After 15 seconds, the pyridine addition was started at a flow rate of exactly 4 ml/min. Throughout the reaction the flow rates of the chlorine and pyridine were maintained precisely at constant rates, and the temperature of the exothermic reaction was held between 40—50°C by occasional application of an ice bath. Initial precipitation of the product was observed between 4 and 5 minutes of reaction. At the 8 minute point (when 31 ml of pyridine had been added) the pyridine addition rate was increased to 8 ml/min. for one minute. The addition of the entire 39 ml of pyridine was thus completed at the 9 minute point. The chlorine flow was continued for 30—45 seconds until a dramatic color change from dark brown to orange was observed. This color change serves as a visual endpoint for the reaction; as soon as it is observed, the chlorine tube must be removed from the solution immediately. In this instance, the reaction mixture was allowed to stir for an additional 5 minutes, and the color reverted from orange to brown. The endpoint color was restored by reintroduction of the chlorine tube for a short period, 10—20 seconds. The reaction mixture was then cooled to 0—5°C by means of an ice bath, and the precipitated product was collected by suction filtration. The product was then washed with 100 ml ice cold acetonitrile, and vacuum dried. TCNQ was obtained as 21.3 g (87% of theoretical yield) of orange-bronze crystals, m.p. 285—288°C, purity 99+%. Analysis calculated for $C_{12}H_4N_4$: C, 70.58; H, 1.97; N, 27.44. Found: C, 70.57; H, 1.93; and N, 27.33.

Industrial Applicability

This invention is, by its nature, one that owes its success to a very specific and stringently controlled set of reaction conditions. Thus, fairly minor deviations in the procedure, other than those which may be necessary for scale-up of the reaction, are likely to be detrimental. However, some variations in the major reaction parameters are permissible.

By "substituted 1,4-bis(dicyanomethylene)cyclohexane" is meant a BDCC compound which is substituted at one or more of the positions on the hexane ring. The prior art, for example, U.S. 3,115,506, issued December 24, 1963 to D. S. Acker et al., and U.S. 3,504,001, issued March 31, 1970, to E. L. Martin, describe the preparation of a variety of substituted TCNQ compounds by oxidation of the corresponding BDCC derivatives, as well as by other synthetic routes. In each instance, the substituted TCNQ compound can be prepared by the halogenation and dehydrohalogenation of the corresponding substituted BDCC compound.

Nitrile solvents similar to acetonitrile, (e.g., butyronitrile) are acceptable, but more expensive. Other solvents have been tried, and found unsuccessful.

Substituted pyridines, or pyridine analogs such as quinoline could be used in place of the pyridine reagent, but would, once again, be more costly. It is important to use an aromatic amine such as pyridine, because aliphatic amines react with TCNQ.

Lower temperatures are less desirable, while higher temperatures provide no advantage. The reaction can be carried out as described at temperatures between 25°C and 75°C.

Reaction times between 5 and 30 minutes are most useful. There is little need to utilize shorter reaction times, and longer reaction times result in loss of the endpoint color change.

As the foregoing discussion indicates, the specific simultaneous addition procedure described herein has been found to afford the maximum yield and purity of TCNQ, and even minor variations can cause substantial reductions in yield and/or purity. At the same time, it will be appreciated that the process of this invention lends itself readily to automation such as microprocessor-based, continuous, real-time analysis and control.

Under such circumstances, or other circumstances within the scope and spirit of this invention, the chlorine and amine addition rates need not be constant, or even linear, so long as the amine addition rate is adjusted to closely track the chlorine addition rate and, preferably, chlorine is maintained in slight excess in the reaction mixture.

**Claims**

1. A process for the halogenation and dehydrohalogenation of a substituted or unsubstituted 1,4-bis(dicyanomethylene)cyclohexane to the corresponding 7,7,8,8-tetracyanoquinodimethane by reacting chlorine and an aromatic amine with the 1,4-bis(dicyanomethylene)cyclohexane in a substantially 4:2:1 amine:chlorine:1,4-bis(dicyanomethylene)cyclohexane molar ratio, comprising simultaneously adding the chlorine and aromatic amine at substantially stoichiometric ratios to a reaction mixture containing the 1,4-bis(dicyanomethylene)cyclohexane, while maintaining chlorine in slight stoichiometric excess.

2. A process according to Claim 1 wherein chlorine is maintained in slight stoichiometric excess by slightly delaying initiation of amine addition.

3. A process according to either one of Claims 1 and 2 which further comprises

a) increasing the addition of the amine beyond a stoichiometric rate, when the reaction is 70—90% complete, so that a stoichiometric quantity of the amine is added prior to reaction completion; and

b) stopping addition of the amine substantially immediately upon addition of a stoichiometric quantity of the amine to the reaction mixture; followed by

c) stopping addition of chlorine substantially immediately upon stoichiometric completion of the reaction.

4. A process according to any of the preceding claims in which the aromatic amine is pyridine.

5. A process according to any of the preceding claims in which stoichiometric completion of the reaction is determined by change of color of the reaction mixture.

6. A process according to any of the preceding claims in which the reaction mixture further contains acetonitrile as a solvent.

**Patentansprüche**

1. Ein Verfahren zur Halogenierung und Dehydrohalogenierung eines substituierten oder unsubstituierten 1,4-Bis(dicyanomethylen)cyclohexans zum entsprechenden 7,7,8,8-Tetracyanochinodimethan durch Umsetzung von Chlor und einem aromatischen Amin mit dem 1,4-Bis(dicyanomethylen)cyclohexan in einem Molverhältnis von Amin:Chlor: 1,4-Bis(dicyanomethylen)cyclohexan, das im wesentlichen 4:2:1 beträgt, umfassend die gleichzeitige Zugabe des Chlors und des aromatischen Amins in im wesentlichen stöchiometrischen Verhältnissen zu einem das 1,4-Bis(dicyanomethylen)cyclohexan enthaltenden Reaktionsgemisch, während ein geringer stöchiometrischer Überschuß von Chlor aufrechterhalten wird.

2. Ein Verfahren nach Anspruch 1, worin der geringe stöchiometrische Überschuß von Chlor durch geringfügige Verzögerung des Beginns der Aminzugabe aufrechterhalten wird.

3. Ein Verfahren nach Anspruch 1 oder 2, welches außerdem

a) die Erhöhung der Zugabe des Amins über ein stöchiometrisches Ausmaß, sobald die Umsetzung zu 70—90% abgelaufen ist, so daß eine stöchiometrische Menge des Amins vor Beendigung der Umsetzung zugesetzt wird; und

b) das Unterbrechen der Zugabe des Amins im wesentlichen sogleich nach Zugabe einer stöchiometrischen Menge des Amins zu dem Reaktionsgemisch; gefolgt von

c) der Unterbrechung der Zugabe von Chlor im wesentlichen sogleich nach stöchiometrischer Beendigung der Umsetzung umfaßt.

4. Ein Verfahren nach einem der vorhergehenden Ansprüche, in welchem das aromatische Amin Pyridin ist.

5. Ein Verfahren nach einem der vorhergehenden Ansprüche, in welchem die stöchiometrische Beendigung der Reaktion durch Farbänderung des Reaktionsgemisches bestimmt wird.

6. Ein Verfahren nach einem der vorhergehenden Ansprüche, in welchem das Reaktionsgemisch außerdem Acetonitril als ein Lösungsmittel enthält.

**Revendications**

1. Procédé pour l'halogénation et la déhydrohalogénation d'un 1,4-bis (dicyanométhylène)cyclohexane substitué ou non substitué en le 7,7,8,8-tétracyanoquinodiméthane correspondant par réaction du chlore et d'une amine aromatique sur le 1,4-bis(dicyanométhylène)cyclohexane selon un rapport molaire amine:chlore: 1,4-bis (dicyanométhylène)cyclohexane pratiquement égal à 4:2:1, consistant à ajouter simultanément le chlore et l'amine aromatique, en quantités pratiquement stoechiométriques, au mélange réactionnel contenant le 1,4-bis (dicyanométhylène)cyclohexane tout en maintenant le chlore en léger excès stoechiométrique.

2. Procédé selon la revendication 1, dans lequel on maintient le chlore en léger excès stoechiométrique en retardant légèrement le début de l'addition de l'amine.

3. Procédé selon l'une ou l'autre des revendications 1 ou 2, consistant en outre:

a) à augmenter l'addition de l'amine au-delà d'un taux stoechiométrique, quand la réaction est achevée à 70—90%, de façon à ajouter avant la fin de la réaction une quantité stoechiométrique de l'amine; et

b) à arrêter l'addition de l'amine presque immédiatement après addition de la quantité stoechométrique de l'amine au mélange réactionnel; puis

c) à arrêter l'addition du chlore presque immédiatement après achèvement de la réaction stoechiométrique.

4. Procédé selon l'une quelconque des revendications précédentes, dans lequel l'amine aromatique est la pyridine.

5. Procédé selon l'une quelconque des revendications précédentes, dans lequel la fin de la réaction stoechométrique est déterminée par un changement de couleur du mélange réactionnel.

6. Procédé selon l'une quelconque des revendications précédentes, dans lequel le mélange réactionnel contient en outre de l'acétonitrile en tant que solvant.

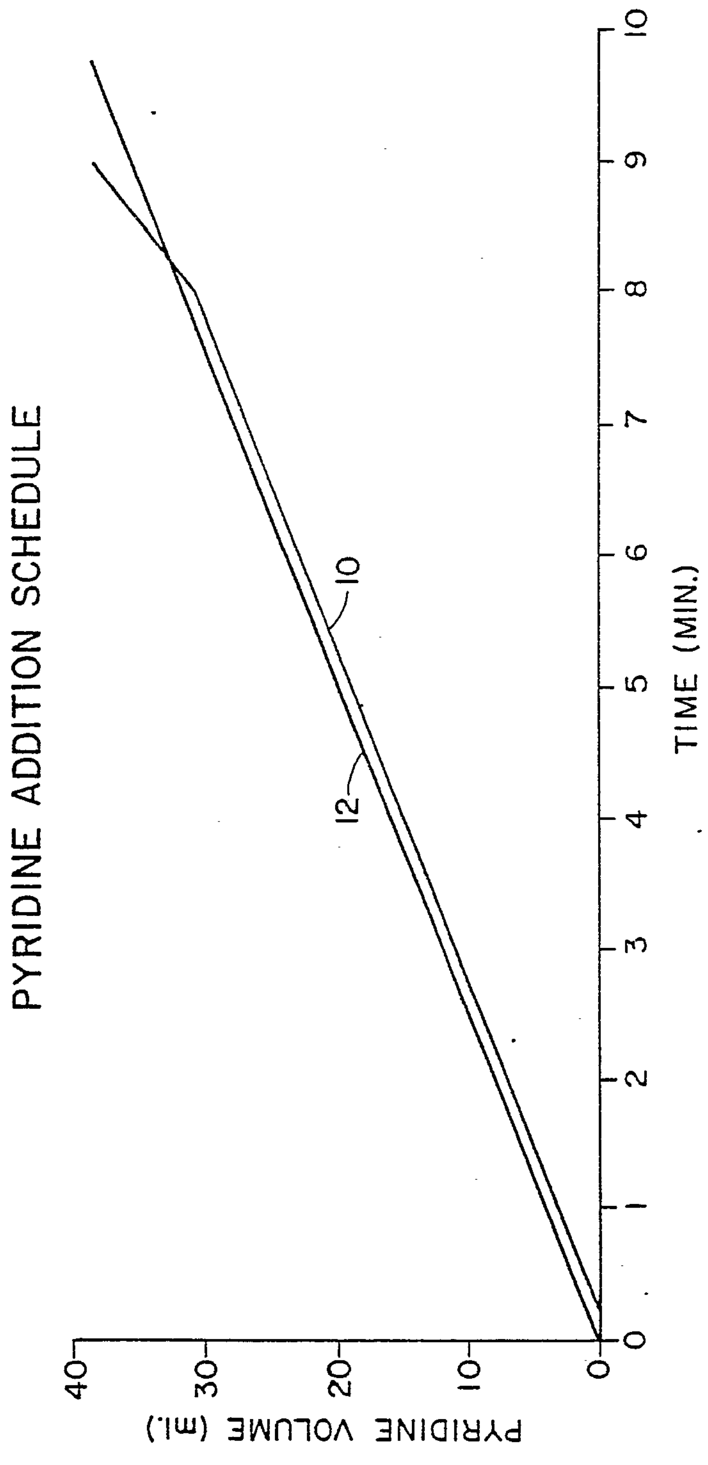

PYRIDINE ADDITION SCHEDULE
PYRIDINE VOLUME (ml.)
0
10
20
30
40
TIME (MIN.)
0
1
2
3
4
5
6
7
8
9
10
10
12